(19) Europäisches Patentamt
European Patent Office
Office européen des brevets

(11) **EP 1 838 861 B1**

(12) **EUROPÄISCHE PATENTSCHRIFT**

(45) Veröffentlichungstag und Bekanntmachung des Hinweises auf die Patenterteilung:
**09.06.2010 Patentblatt 2010/23**

(21) Anmeldenummer: **06703801.8**

(22) Anmeldetag: **10.01.2006**

(51) Int Cl.:
***C12P 7/64*** (2006.01)

(86) Internationale Anmeldenummer:
**PCT/EP2006/000120**

(87) Internationale Veröffentlichungsnummer:
**WO 2006/077022 (27.07.2006 Gazette 2006/30)**

(54) **HERSTELLUNG VON MONOGLYCERIDEN AUS TRIGLYCERIDEN DURCH ALKOHOLYSE UNTER VERWENDUNG DER THERMOMYCES LANUGINOSUS LIPASE, WELCHE DURCH ALKALISCHE SALZE AKTIVIERT WIRD**

PRODUCTION OF MONOGLYCERIDES FROM TRIGLYCERIDES BY ALCOHOLYSIS EMPLOYING THERMOMYCES LANUGINOSUS LIPASE WHICH IS ACTIVATED BY ALKALINE SALTS

PRODUCTION DE MONOGLYCERIDES A PARTIR DE TRIGLYCERIDES PAR ALCOOLYSE, A L'AIDE DE LA LIPASE DE THERMOMYCES LANUGINOSUS ACTIVEE PAR DES SELS ALCALINS

(84) Benannte Vertragsstaaten:
**AT DE ES FR GB**

(30) Priorität: **19.01.2005 DE 102005002711**

(43) Veröffentlichungstag der Anmeldung:
**03.10.2007 Patentblatt 2007/40**

(73) Patentinhaber: **Cognis IP Management GmbH 40589 Düsseldorf (DE)**

(72) Erfinder:
• **SCHÖRKEN, Ulrich**
**40591 Düsseldorf (DE)**
• **BOTH, Sabine**
**41470 Neuss (DE)**
• **BONGARDT, Frank**
**40670 Meerbusch (DE)**
• **STUHLMANN, Diana**
**40627 Düsseldorf (DE)**

(56) Entgegenhaltungen:
EP-A- 1 300 470      WO-A-90/04033
WO-A-91/16442      WO-A-2006/077023
FR-A- 2 772 391

• **PATENT ABSTRACTS OF JAPAN Bd. 015, Nr. 440 (C-0883), 11. November 1991 (1991-11-11) & JP 03 187385 A (MEITO SANGYO KK), 15. August 1991 (1991-08-15) in der Anmeldung erwähnt**
• **PATENT ABSTRACTS OF JAPAN Bd. 009, Nr. 247 (C-307), 3. Oktober 1985 (1985-10-03) & JP 60 102192 A (MEITO SANGYO KK), 6. Juni 1985 (1985-06-06)**
• **PATENT ABSTRACTS OF JAPAN Bd. 015, Nr. 297 (C-0854), 29. Juli 1991 (1991-07-29) & JP 03 108489 A (MEITO SANGYO KK), 8. Mai 1991 (1991-05-08) in der Anmeldung erwähnt**
• **PATENT ABSTRACTS OF JAPAN Bd. 015, Nr. 289 (C-0852), 23. Juli 1991 (1991-07-23) & JP 03 103499 A (MEITO SANGYO KK), 30. April 1991 (1991-04-30) in der Anmeldung erwähnt**

EP 1 838 861 B1

## Beschreibung

### Gebiet der Erfindung

Die Erfindung befindet sich auf dem Gebiet der Glyceride und betrifft ein Verfahren zur Herstellung von Monoglyceriden durch enzymatische Katalyse.

### Stand der Technik

In der chemischen und biochemischen Synthese werden vermehrt Enzyme als Katalysatoren eingesetzt. So werden in vielen Fällen aufgrund der oft milderen Reaktionsbedingungen bereits in großtechnischen Verfahren Hydrolasen, speziell Lipasen (EC 3.1.1.3), zur Fettspaltung eingesetzt.

Diese Enzyme werden von unterschiedlichen Mikroorganismen produziert. Zur Isolierung der Enzyme folgt nach der Fermentation der Mikroorganismen ein aufwendiges Reinigungsverfahren.

Der Effektivität dieser Katalysatoren stehen oftmals die hohen Kosten der Produktion und der Isolierung gegenüber, so dass Forschungsgrappen immer wieder bestrebt sind, die Ausbeuten an Enzymen zu erhöhen oder die Produktivität der Enzyme zu steigern.

Die klassische chemische Methode der Monoglyceridherstellung verläuft über eine basekatalysierte Glycerolyse von Triglyceriden, wobei typischerweise eine Ausbeute von 40 - 60 % Monoglycerid bezogen auf die Gesamtglyceride erhalten werden. Eine weitere Anreicherung bis auf > 90 % Monoglyceridgehalt erfolgt über physikalische Trennmethoden wie Molekulardestillation oder Kristallisation.

Enzymatisch sind in der Literatur verschiedene Routen beschrieben worden, die zur Herstellung von Monoglyceriden geeignet sind: 1) die enzymatische Synthese ausgehend von Fettsäure und Glycerin; 2) die enzymatische Glycerolyse ausgehend von Triglycerid und Glycerin, die dem chemischen Verfahren entspricht; 3) die 1,3-regioselektive Hydrolyse oder Alkoholyse von Triglycerid. Zusammenfassungen zu den Verfahren finden sich z.B. in ((a) Recent Res. Devel. Oil Chem., 3 (1999), 93-106; (b) Hydrolases in Organic Synthesis, Wiley-VCH (1999), eds. Bornscheuer & Kazlaukas).

Die enzymatische Synthese von Monoglyceriden funktioniert nur gut unter Entfernung von Wasser aus dem Reaktionsgleichgewicht, was durch Zugabe von Molekularsieb oder durch eine Reaktion unter Vakuum erreicht wird. Zudem werden Lösungsvermittler für eine gute Synthese benötigt ((a) Recent Res. Devel. Oil Chem., 3 (1999)). Daher ist die enzymatische Synthese von Monoglyceriden keine kostengünstige Alternative zum chemischen Verfahren. Die enzymatische Glycerolyse führt zu ähnlichen Gleichgewichtseinstellungen wie die chemische Glycerolyse zur Monoglyceridherstellung. Für eine Synthese von angereicherten Monoglyceriden (Gehalt > 60 %) ist daher auch eine Anreicherung über Destillation oder Kristallisation notwendig. Damit ist auch dieses Verfahren keine kostengünstige Alternative zum chemischen Verfahren.

WO9013656 und WO9004033 (Enzytech Inc.) sowie US5935828 und US5316927 (Opta Food Ingredients Inc.) beschreiben die Herstellung von Monoglyceriden über enzymatische Alkoholyse mit verschiedenen Alkoholen und wenig Wasser im Ansatz. Eingesetzt werden Lipasen in Pulverform oder immobilisiert. In den Beispielen werden Lipasen in etwa 20 Gew.% bezogen auf das Triglycerid eingesetzt und die Alkoholkomponente im 20fachen Überschuß.

Die W09116441, W09116442 und US5116745 beschreiben Verfahren, bei dem in Anwesenheit eines Lösungsmittels, eines Alkohols und eines wässrigen Puffers eine gemischte regioselektive Alkoholyse und Hydrolyse zu 1,2-Diglyceriden und 2-Monoglyceriden unter Verwendung von Lipasen erfolgt.

EP407959 beschreibt ein Verfahren zur Herstellung von Monoester durch eine thermostabile immobilisierte Lipase in Gegenwart von sekundären oder tertiären Alkoholen als Lösungs vermittler.

WO0206505 (Nippon Suisan Kaisha Ltd) beschreibt die regioselektive Alkoholyse mit immobilisierter Lipase bei hohem Alkoholüberschuß und hoher Enzymeinsatzkonzentration, gefolgt von einer Wiederveresterung des Monoglycerids.

JP60102192 und JP03187385 (Meito Sangyo Co. Ltd.) beschreiben die Hydrolyse von Triglyceriden mit alkaliner Lipase unter Zugabe von alkalischen Salzen. Es handelt sich um eine Lipase, die nur unter alkalischen Bedingungen aktiv ist.

JP03103499 (Meito Sangyo Co. Ltd.) beschreibt die regioselektive Alkoholyse von PUFA-Triglyceriden mit Isobutanol in Anwesenheit einer alkalischen Lipase.

Die enzymatische Herstellung von Monoglyceriden wurde schon vielfach beschrieben, doch in allen oben genannten Dokumenten werden Lösungsmittel benötigt, das Reaktionswasser muss aufwendig entfernt werden oder die Lipasen sind sehr speziell bzw. immobilisiert. Durch die teilweise niedrigen Reaktionsraten im Vergleich zur klassisch chemischen Synthese werden lange Reaktionszeiten und damit hohe Kapazitätsauslastungen oder hohe Konzentrationen an zu reagierenden Alkohol oder an Lipase benötigt um eine höhere Ausbeute an gewünschten Monoglyceriden zu erhalten. Auch wenn kostengünstige Lipasen zum Einsatz kommen, führt dass zu Verfahren, die einen industriellen Prozess schon allein aus Kostengründen unmöglich machen.

Die Aufgabe der vorliegenden Erfindung hat nun darin bestanden, eine kostengünstige Variante zu finden um die Ausbeute an Monoglyceriden aus Polyolestern wie beispielsweise Triglyceriden bei enzymatischen Alkoholysen zu erhöhen und den Gehalt an Enzym möglichst gering zu halten.

**Beschreibung der Erfindung**

Gegenstand der Erfindung ist ein Verfahren zur Herstellung von Monoglyceriden, bei dem Triglyceride in Anwesenheit von linearen oder verzweigten Alkoholen mit einer Anzahl an Kohlenstoffatomen von 1 - 8 C-Atomen mit einer Lipase aus Thermomyces lanuginosus enzymatisch umgesetzt werden, welche durch Zugabe von alkalischen Salzen aktiviert wird.

Überraschenderweise wurde gefunden, dass die Zugabe von alkalischen Salzen die Lipase aus Thermomyces lanuginosus aktivieren kann und dadurch im Vergleich zu bekannten Verfahren eine erhöhte Ausbeute an Monoglyceriden bei der Alkoholyse von Triglyceriden erreicht werden kann. Bei dem erfindungsgemäßen Verfahren wird ein Triglycerid in Anwesenheit eines Alkohols in ein 2-Monoglycerid und zwei Fettsäureester gespalten.

Die Reaktion kann durch den Einsatz von geringen Mengen an der Lipase sehr kostengünstig durchgeführt werden. Die Reaktion wird direkt mit dem Enzymkonzentrat unter Zugabe von einem alkalischen anorganischen Salz durchgeführt, dass eine starke Aktivierung des Enzyms bewirkt. Hierdurch wird eine gute Umsetzung bei geringer Enzymdosierung auch ohne Stabilisierung des Enzyms durch Immobilisation erreicht. Eine Zugabe von Lösungsmitteln ist nicht notwendig.

Die Alkoholyse wird bei Temperaturen von 10° bis 40° C, bevorzugt bei 10° bis 30 ° C und besonders bevorzugt bei einer Temperatur von 15° bis 25° C durchgeführt.

Die Reaktion wird bei einem Wassergehalt von 0,1 - 10 Gew.-%, bevorzugt 0,1- 5 Gew.-% und besonders bevorzugt 0,1-2 Gew.%, bezogen auf die Menge an Triglycerid durchgeführt, wobei der Wassergehalt der flüssigen Enzympraparation mit einberechnet ist. Die Reaktion funktioniert auch bei höheren Wassergehalten, allerdings ist dann der Gehalt an gebildeter freier Fettsäure erhöht. Die freie Fettsäure ist nicht gewünscht, da sie in der destillativen Trennung von Ester und Glyceridgemisch eine Rückveresterung eingehen kann und damit die Ausbeute an Monoglycerid reduziert.

Die Reaktionszeit beträgt erfindungsgemäß bevorzugt 12 - 48 h in Abhängigkeit der eingesetzten Enzymkonzentration. Bevorzugt werden alle Reaktanden vermischt und die Reaktion durch Zugabe der Enzympräparation gestartet.

Die Zugabe der Alkoholkomponente, bevorzugt Methanol und/oder Methanol, Ethanol bevorzugt, erfolgt entweder komplett am Anfang oder über den Reaktionszeitraum dosiert.

Die eingesetzte Alkoholmenge ist variabel, Minimum 2 Mol Alkohol auf 1 Mol Öl, Maximum 75 Gew.-% Alkohol und 25 Gew.-% Öl im Ansatz.

In einem weiteren Schritt des erfindungsgemäßen Verfahrens wird die Lipase durch Hitze, deaktiviert und anschließend die präzipitierte Lipase gegebenenfalls abfiltriert wobei neben der präzipitierten Lipase die Entfernung von Additiven oder Formuhmmgsbestandteilen der eingesetzten Enzympräparation erreicht werden kann. Gleichzeitig kann die Alkoholkomponente über Destillation beispielweise bei 80° C und 100 mbar Vakuum entfernt werden und ein Gemisch aus Alkylester und Monoglycerid wird erhalten. Die Alkylester und Monoglycerid können in einem nächsten Schritt destillativ getrennt werden, beispielsweise über einen Dünnschichtverdampfer oder über eine Kolonne. Reaktionsbedingungen liegen beispielsweise bei 175° C und 0,3 mbar Vakuum. Das Monoglycerid verbleibt im Sumpf.

Für das erfindungsgemäße Verfahren hat sich die Lipase von *Thermomyces lanuginosus* (Hersteller Novozymes, Bezeichnung Lipozyme TL1001 oder Lipolase 100 EX) als besonders geeignet erwiesen. Experimentelle Daten haben gezeigt, dass die Zugabe geringer Mengen alkalischer anorganischer Salze die Enzymaktivität dieser Lipase drastisch erhöht. Inbesondere wird nicht immobilisierte Lipase durch die alkalischen Salze aktiviert.

Bevorzugt wird eine Einsatzkonzentration von 0,05 - 2 % der kommerziell erhältlichen Flüssigpräparation in Bezug auf die Menge an eingesetztem Triglycerid eingesetzt. Diese kommerziell erhältlichen Enzym-Flüssigpräparate weisen im Durchschnitt eine Enzymaktivität von 100.000 U/ml. Eine Enzymeinheit U (enzyme units) ist definiert als Enzymmenge, die pro Minute ein micromol Substrat umsetzt.

Für das erfindungsgemäße Verfahren werden zur Aktivierung der Lipase bevorzugt alkalische anorganische Salze verwendet, die ausgewählt sind aus der Gruppe, die gebildet wird von Hydroxiden, Carbonaten und Phosphaten des Natriums, Kaliums, Calciums, Magnesiums und Ammoniums, vorgelöst in Wasser. Die Menge an alkalischen anorganischen Salzen zur Aktivierung der Lipase beträgt erfindungsgemäß zwischen 0,00001 und 1 Gew.-%, bevorzugt zwischen 0,0001 und 0,2 Oew.-% bezogen auf die Menge an Triglycerid. Die Einsatzmenge an basischem Additiv ist abhängig von der Menge an eingesetzter Enzym-Flüssigpräparation, die gepuffert ist sowie von der Stärke der Base. Bei Einsatz von NaOH und < 0,5 % Enzym-Flüssigpräparation ist die Einsatzkonzentration im unteren Konzentrationsbereich, wobei bei Einsatz von $Na_2CO_3$ und 2 % Enzym-Flüssigpräparation die Menge an basischem Additiv am oberen Konzentrationsbereich liegt.

Überraschenderweise wurde die stärkste Aktivierung der *Thermomyces lanuginosus* Lipase erreicht, wenn zur kommerziell erhältlichen Enzym-Flüssigpräparation Salze wie z.B. Trinatriumphosphat, Natriumcarbonat, Natriumhydroxid oder Ammoniumhydroxid in Mengen von 0,0001 - 0,2 Gew.-% (bezogen auf den Triglyceridgehalt) gegeben wurden. Überraschenderweise wurde eine schnellere Monoglycerid Synthesegeschwindigkeit erreicht als mit der Lipase adsorbiert auf Polypropylen. Die Aktivierung der Lipase ist so stark, dass-sie nicht alleine durch den pH-Shift im Reaktionsmedium erklärt werden kann. Wird die *Thermomyces lanuginosus* Lipase immobilisiert unter den gleichen Bedingungen eingesetzt, ist keine ähnlich starke Aktivierung über Salzzugabe zu erkennen. Diese starke Aktivierung ist sehr überraschend, da allgemein akzeptiert ist, dass im wasserarmen Medium nur mit Lipasen, die an einen Träger gebunden sind, eine hohe Aktivität erreicht werden kann. Durch diese starke Aktivierung kann auf aufwendige Immobilisationsprozesse verzichtet werden und es führt zu einem simplen Anlagenkonzept.

Eine Messung des pH-Wertes des umgesetzten Produktgemisches zeigt zudem, dass der pH-Wert im neutralen bis schwach sauren liegt, was eine Enzymaktivierung alleine über pH-Shift unwahrscheinlich macht.

Bei dem erfindungsgemäßen Verfahren werden bevorzugt Triglyceride aus Fetten und Ölen eingesetzt, die einen hohen Anteil an einfach und/oder mehrfach ungesättigter Fettsäuren haben und ausgewählt sind aus der Gruppe, die gebildet wird aus Sonnenblumenöl, Rapsöl, Distelöl, Sojaöl, Leinöl, Erdnussöl, Talge, Olivenöl, Rizinusöl, Palmöl und Altöle wie beispielsweise gebrauchtes Frittierfett.

Erdnussöl enthält durchschnittlich (bezogen auf Fettsäure) 54 Gew.-% Ölsäure, 24 Gew.-Linolsäure, 1 Gew.-% Linolensäure, 1 Gew.-% Arachinsäure, 10 Gew.-% Palmitinsäure, sowie 4 Gew.-% Stearinsäure. Der Schmelzpunkt beträgt 2 bis 3 ˚C.

Leinöl enthält typischerweise 5 Gew.-% Palmitin-, 4 Gew.-% Stearin-, 22 Gew.-% Öl-, 17 Gew.-% Linol- und 52 Gew.-% Linolensäure. Die Iodzahl liegt im Bereich von 155 bis 205, Die Verseifungszahl ist 188 bis 196 und der Schmelzpunkt liegt bei etwa - 20 ˚C.

Olivenöl enthält überwiegend Ölsäure. Palmöl enthält als Fettsäurekomponenten etwa 2 Gew.-% Myristin-, 42 Gew.-% Palmitin-, 5 Gew.-% Stearin-, 41 Gew.-% Öl-, 10 Gew.-% Linolsäure.

Rapsöl enthält als Fettsäurekomponenten typischerweise etwa 48 Gew.-% Erucasäure, 15 Gew.-% Ölsäure, 14 Gew.-% Linolsäure, 8 Gew.-% Linolensäure, 5 Gew.-% Icosensäure, 3 Gew.-% Palmitinsäure, 2 Gew.-% Hexadecensäure und 1 Gew.-% Docosadiensäure. Rapsöl aus neuer Züchtung ist bezüglich der ungesättigten Anteile angereichert. Typische Fettsäureanteile sind hier Erucasäure 0,5 Gew.%, Ölsäure 63 .Gew.%, Linolsäure 20 Gew.-%, Linolensäure 9 Gew.%, Icosensäure 1 Gew.%, Palmitinsäure 4 Gew.%, Hexadecensäure 2 Gew.-% und Docosadiensäure 1 Gew.-%.

Ricinusöl besteht zu 80 bis 85 Gew.-% aus dem Glycerid der Ricinolsäure, daneben sind zu etwa 7 Gew.-%_ Glyceride der Öl-, zu 3 Gew.-% Glyceride der Linol- und zu etwa 2 Gew.-% die Glyceride der Palmitin-und der Stearinsäure enthalten.

Sojaöl enthält zu 55 bis 65 Gew.-% der Gesamtfettsäuren mehrfach ungesättigte Säuren, insbesondere Linol- und Linolensäure. Ähnlich ist die Situation beim Sonnenblumenöl, dessen typisches Fettsäurespektrum, bezogen auf Gesamtfettsäure wie folgt aussieht: ca. 1 Gew.-% Myristin-, 3 bis 10 Gew.-% Palmitin-, 14 bis 65 Gew.-% Öl- und 20 bis 75 Gew.-% Linolsäure.

Alle obigen Angaben über die Fettsäureanteile in den Triglyceriden sind bekanntermaßen abhängig von der Qualität der Rohstoffe und können daher zahlenmäßig schwanken:

Die Fettsäurezusammensetzung in dem Gemisch ergibt sich aus der jeweiligen nativen Fettsäurezusammensetzung des verwendeten Pflanzenöles sowie der jeweiligen Qualität des Rohstoffes aus dem die Methyl- und/oder Ethylester in bekannter Art hergestellt werden.

Für das erfindungsgemäße Verfahren werden bevorzugt lineare oder verzweigte Alkohole als Alkoholkomponenten eingesetzt, die eine Anzahl an Kohlenstoffatomen von 1 - 8 C-Atomen aufweisen. Diese können bevorzugt primäre oder sekundäre Alkohole darstellen. Als bevorzugte Alkoholkomponente werden Ethanol oder 1-Propanol eingesetzt. Der Gehalt an Alkohol beträgt bevorzugt 10 bis 75 Gew.-% bezogen auf das eingesetzte Triglycerid, bevorzugt werden 15 bis 40 Gew.-% eingesetzt. Der Monoglyceridgehalt ist abhängig von der eingesetzten Alkoholmenge.

In einer bevorzugten Ausführungsform des erfindungsgemäßen Verfahrens kann der Alkohol entfernt werden, bevorzugt über Destillation. Durch diesen zusätzlichen Verfahrensschritt werden Monoglyceride im Gemisch mit Alkylestern erhalten, die in der Mischung einsetzbar sind als Schmiermitteladditiv, als Additiv für Kraftstoffe oder als emulgierender Bestandteil in Nahrungsmitteln, kosmetischen und/oder pharmazeutischen Formulierungen.

In einer weiteren bevorzugten Ausführungsform des erfindungsgemäßen Verfahrens können die Alkylester von den Monoglyceriden getrennt werden, bevorzugt über Destillation. Durch diesen zusätzlichen Verfahrensschritt werden Monoglyceride erhalten, die einsetzbar sind als Schmiermitteladditiv, als Additiv für Kraftstoffe oder als emulgierender Bestandteil in Nahrungsmitteln, kosmetischen und/oder pharmazeutischen Formulierungen.

Der Einsatz von verschiedenartigen Additiven für Kraftstoffe ist literaturbekannt. Monoglyceride und andere partiell veresterte oder veretherte Polyole (z.B. auch Glykolinonoester) werden als Dieseladditiv zugesetzt, da sie eine gute Schmierwirkung aufweisen. Patentanmeldungen, die solche Additive beschreiben sind z.B. EP0721492 (Infineum USA L.P.), WO0119941 (Fina Research S.A.) oder WO0063322 (Pure Fuels USA Inc.). Insbesondere Glyceridmischungen mit einem hohen Anteil an Monoglycerid zeigen gute Schmiereigenschaften. So konnte gezeigt werden, dass auch die nach dem erfindungsgemäßen Verfahren hergestellten Monoglyceride als Kraftstoffadditive in Dieselkraftstoff einsetzbar sind und gute Schmiereigenschaften zeigen.

Mit dem erfindungsgemäßen Verfahren lässt sich die regiospezifische Fettsäurezusammensetzung der natürlich vorkommenden Öle nutzen. Die Monoglyceridfraktion enthält hauptsächlich die Fettsäurezusammensetzung, die in der 2-Position der Öle zu finden ist. Bei den meisten natürlich vorkommenden Ölen sind die höher ungesättigten Fettsäuren bevorzugt in der 2-Position gebunden. Dadurch lassen sich z.B. ausgehend von Sonnenblumen- oder Distelöl Monoglyceride mit einem hohen Gehalt an Linolsäure herstellen. Diese Monoglyceride haben einen erniedrigten Erstarrungspunkt, der insbesondere für die Anwendung von Monoglyceriden als Dieseladditiv wichtig ist. Basierend auf Palmöl lässt sich z.B. ein Monoglycerid mit einem hohen Gehalt an Ölsäure gewinnen, was insbesondere für den Einsatz in Lebensmitteln oder Kosmetika geeignet ist.

Der Einsatz von Monoglyceriden im Nahrungsmittelbereich ist ebenfalls literaturbekannt. Nach EG Richtlinien müssen der Gehalt von Mono- und Diglycerid mindestens 70 % betragen, die Säurezahl darf 6 nicht überschreiten, das Produkt darf maximal 7 % freies Glycerin und 2 % Wasser enthalten. Über das enzymatische Verfahren gekoppelt mit destillativer Abtrennung der Fettsäureester werden Monoglyceride mit > 90 % Mono- und Diester sowie > 70 % Monoestergehalt erreicht. Die Maximalwerte für Wassergehalt, freies Glycerin und Säurezahl werden deutlich unterschritten. Damit ist der Einsatz von Monoglyceriden hergestellt nach dem erfindungsgemäßen Verfahren im Nahrungsmittelbereich gegeben.

Der Einsatz von Monoglyceriden in kosmetischen oder pharmazeutischen Formulierungen ist ebenfalls literaturbekannt. Die erfindungsgemäß hergestellten Monoglyceride mit > 90 % Mono- und Diester sowie > 70 % Monoestergehalt können eingesetzt werden als W/O-Emulgatoren, Coemulgatoren, Rückfettungskomponenten, Konsistenzfaktoren oder Konsistenzgeber in Cremes, Lotionen, Salben, Tensidpräparaten sowie in kosmetischen und pharmazeutischen Wasser in Öl (W/O) und Öl in Wasser (O/W) Emulsionen.

## Beispiele

### Beispiel 1: Regioselektive Alkoholyse mit verschiedenen Enzymene in freier und immobilisierter Form

16 Ansätze bestehend aus 20 g Rapsöl und 2,5 g Ethanol wurden in Bechergläser ausgestattet mit Magnetrührern vorgelegt. Unter Rühren wurden in die Ansätze 1-9, sowie 15 + 16 jeweils 0,25 g Wasser zugegeben und in die Ansätze 10-14 wurden jeweils 0,5 g Wasser zugegeben. Anschliessend wurden Lipasen in freier sowie immobilisierter Form wie in der Tabelle unten aufgelistet zugegeben. Die Ansätze wurden unter Rühren für 24 h inkubiert, wobei nach 5 h weitere 2,5 g Ethanol zugegeben wurden. Die Alkoholyse der Ansätze 1-14 wurde auf einer Multirührplatte bei Raumtemperatur durchgeführt. Die Ansätze 15 + 16 wurden auf einem Schüttler bei 45 ˚C inkubiert. Nach 24 h wurden Proben entnommen und der Gehalt an Glyceriden und Ethylestern wird gaschromatographisch analysiert. Die Auswertung erfolgte über Flächenprozent. Geringe Anteile an gebildeter Fettsäure sind in der Fläche der Ethylester enthalten.

Die Immobilisate der Ansätze 1 - 3, sowie 15 + 16 wurden direkt vom Hersteller in immobilisierter Form erworben. Die Immobilisate der Ansätze 4-8 wurden über Adsorption auf Accurel MP 1000 (Membrana) hergestellt. Dazu wurden 1 Accurel MP 1000 für 1 h in 10 ml Ethanol inkubiert. Nach Abdekantieren des Ethanols wurden 10g Wasser und jeweils 0,5 g der Lipasepräparation zugegeben. Das Gemisch wurde über Nacht bei Raumtemperatur inkubiert. Anschliessend wurde das Immobilisat über Filtration separiert und für 24 h auf Papierbögen bei Raumtemperatur getrocknet.

| Ansatz | Enzym | Hersteller | Organismus | Form |
|---|---|---|---|---|
| 1 | 1 g Novozym 435 | Novozymes | C.antarctica B | Immobilisat |
| 2 | 1 g Lipozym RM IM | Novozymes | R.miehei | Immobilisat |
| 3 | 1 g Lipozym TL IM | Novozymes | T.lanuginosus | Immobilisat |
| 4 | 1 g Lipase FAP 15 / MP 1000 | Amano | R.oryzae | Immobilisat |
| 5 | 1 g Lipase A / MP 1000 | Amano | A.niger | Immobilisat |
| 6 | 1 g Lipase M / MP 1000 | Amano | M.javanicus | Immobilisat |
| 7 | 1 g Lipase L115 / MP 1000 | Biocatalysts | Porcine pancreas | Immobilisat |
| 8 | 1 g Lipomod 36 / MP 1000 | Biocatalysts | R.javanicus | Immobilisate |

(fortgesetzt)

| Ansatz | Enzym | Hersteller | Organismus | Form |
|---|---|---|---|---|
| 9 | 0,5 g Lipolase | Novozymes | T.lanuginosus | Frei |
| 10 | 0,5 g Lipase FAP 15 / MP 1000 | Amano | R.oryzae | Frei |
| 11 | 0,5 g Lipase A / MP 1000 | Amano | A.niger | |
| 12 | 0,5 g Lipase M / MP 1000 | Amano | M.javanicus | |
| 13 | 0,5 g Lipase L115 / MP 1000 | Biocatalysts | Porcine pancreas | |
| 14 | 0,5 g Lipomod 36 / MP 1000 | Biocatalysts | R.javanicus | |
| 15 | 1 g Novozym 435 | Novozymes | C.antarctica B | Immobilisat |
| 16 | 1 g Lipozym RM IM | Novozymes | R.miehei | Immobilisat |

| Ansatz | % Ethylester | % Monoglycerid | % Diglycerid | % Triglycerid |
|---|---|---|---|---|
| 1 | 18,2 | 1,4 | 5,0 | 75,4 |
| 2 | 39,3 | 16,2 | 14,5 | 29,5 |
| 3 | 62,7 | 23,5 | 10,9 | 0,5 |
| 4 | 58,5 | 29,6 | 9,6 | 0,0 |
| 5 | 5,2 | 1,6 | 4,6 | 88,6 |
| 6 | 41,7 | 16,5 | 27,7 | 14,1 |
| 7 | 82,4 | 6,8 | 7,0 | 2,9 |
| 8 | 57,7 | 32,7 | 8,3 | 0,0 |
| 9 | 15,9 | 4,1 | 14,8 | 65,2 |
| 10 | 0,0 | 0,0 | 2,1 | 96,2 |
| 11 | 2,0 | 0,4 | 1,6 | 96,0 |
| 12 | 3,4 | 0,0 | 2,4 | 94,2 |
| 13 | 2,2 | 0,4 | 2,3 | 95,1 |
| 14 | 3,3 | 0,0 | 2,8 | 93,9 |
| 15 | 41,0 | 0,0 | 2,2 | 55,8 |
| 16 | 3,7 | 0,0 | 2,3 | 94,0 |

**Beispiel 2: Regioselektive Alkoholyse von Sb-Öl mit nicht immobilisierten Lipasen**

6 Ansätze bestehend aus 40 g Sonnenblumenöl und 10 g Ethanol wurden in Bechergläser ausgestattet mit Magnetrührern vorgelegt. Unter Rühren wurden 0,4 g Wasser zugefügt. In die Ansätze 2, 4 und 6 wurden 40 mg festes $Na_3PO_4$ x 12 $H_2O$ zugegeben. In die Ansätze 1 und 2 wurden 0,4 g Lipolase (*Thermomyces lanuginosus* Lipase Flüssigpräparation), in die Ansätze 3 und 4 wurden 0,4 g Novozym 525 (*Candida antarctica B* Lipase, Flüssigpräparation) und in die Ansätze 5 und 6 wurden 0,4 g Novozym 388 (*Rhizomucor miehei* Lipase Flüssigpräparation) gegeben. Die Alkoholyse wurde auf einer Multirührplatte bei Raumtemperatur durchgeführt. Nach 16 h und 44 h wurden Proben entnommen und der Gehalt an Glyceriden gaschromatographisch analysiert. Auswertung erfolgte über Flächenprozent.

| Ansatz | Dauer | % Ethylester | Gehalt Monoglycerid | Verhältnis Mono-/Di-/Triglycerid |
|---|---|---|---|---|
| 1 | 16 | 0 | 0 % | 0 : 12 : 88 |
| 1 | 44 | 0,7 | 0 % | 0 : 4 : 96 |

(fortgesetzt)

| Ansatz | Dauer | % Ethylester | Gehalt Monoglycerid | Verhältnis Mono-/Di-/Triglycerid |
|--------|-------|--------------|---------------------|----------------------------------|
| 2 | 16 | **55,1** | **26,5 %** | 63 : 33 : 4 |
| 2 | 44 | **61,1** | **23,3 %** | 69 : 31 : 0 |
| 3 | 16 | 0,7 | 0 % | 0 : 2 : 98 |
| 3 | 44 | 2,2 | 0 % | 0 : 4 : 96 |
| 4 | 16 | 0,7 | 0 % | 0 : 2 : 98 |
| 4 | 44 | 2,2 | 0 % | 0 : 4 : 96 |
| 5 | 16 | 7,6 | 0 % | 0 : 4 : 96 |
| 5 | 44 | 4,9 | 1,2 % | 2 : 7 : 91 |
| 6 | 16 | 2,1 | 0 % | 0 : 3 : 97 |
| 6 | 44 | 4,1 | 0,9 % | 1 : 5 : 94 |

Fazit: Lipolase in Anwesenheit eines basischen Salzes zeigte eine signifikante Aktivität (Ansatz 2). Wurde dagegen kein Salz zugegeben, war nur eine sehr schwache Alkoholysereaktion detektierbar.

Eine schwache Aktivität wird mit Novozym 388 detektiert, die aber unabhängig von der Zugabe von basischem Salz ist.

**Beispiel 3: Vergleich der Aktivität von immobilisierter Lipolase und Lipolase Flüssigpräparation**

Verglichen wurden Ansätze, die 0,2 g Lipolase Flüssigpräparation enthielten oder eine entsprechende Menge Lipolase trägergebunden enthielten.

**Immobilisation von Lipolase auf Accurel MP 1000 (Membrana)**: 5 g MP1000 wurden in einen 250 ml Erlenmeyerkolben gegeben und 15 ml Ethanol wurde zugesetzt. Die Mischung wurde 1 h geschüttelt, dann wurde Ethanol abdekantiert. Zum MP1000 wurden 50 g Wasser gegeben. Nach 1 h Rühren wurde das Wasser abdekantiert. 100 ml Phosphatpuffer, 20 mM, pH 6,0 wurde zugegeben und die Immobilisation durch Zugabe von 5 g Lipolase Flüssigpräparation gestartet. Die Ansätze wurden über Nacht bei 8° C gerührt, dann wurde das Enzymimmobilisat abfiltriert. Das Immobilisat wurde über Nacht zwischen Papiertüchern bei Raumtemperatur getrocknet. Das Immobilisat wurde ausgewogen und eine Immobilisatmenge, die 0,2 g Lipolase Flüssigpräparation entspricht, für die Alkoholyse eingesetzt.

**Immobilisation von Lipolase auf Accurel MP 1000 (Membrana) alternativ**: Die Immobilisation erfolgte wie oben beschrieben. Nach Abfiltration des Immobilisats wurden 5 ml einer 200 mM $Na_3PO_4$ Lösung zugegeben. Das komplette Gemisch wurde bei Raumtemperatur unter Vakuum getrocknet. Ziel dieses zusätzlichen Schrittes war es ein bereits alkalisches Immobilisat herzustellen. Das Immobilisat wurde ausgewogen und eine Immobilisatmenge, die 0,2 g Lipolase Flüssigpräparation entspricht, für die Alkoholyse eingesetzt.

**Immobilisation von Lipolase auf Dowex Marathon WBA (Dow Chemicals)**: 200 mg Dowex WBA wurden in einem kleinen Becherglas vorgelegt. 0,2 g Lipolase Flüssigpräparation wurden zupipettiert und mit einer Pipettenspitze gut vermischt. Der Ansatz inkubiert für 2 h unter gelegentlichem Mischen bei Raumtemperatur. Der komplette Ansatz (Dowex + Überstand) wurde für die Transformation eingesetzt.

Parallel durchgeführte Versuche, bei denen nicht gebundene Lipolase über Auswaschen aus dem Immobilisat gewonnen wurde, zeigten, dass etwa 90 % der Lipolase trägergebunden vorliegt.

**Immobilisation von Lipolase auf Duolite A568 (Rohm & Haas)**: 200 mg Duolite A568 wurden in einem kleinen Becherglas vorgelegt. 0,2 g Lipolase Flüssigpräparation wurden zupipettiert und mit einer Pipettenspitze gut vermischt. Der Ansatz inkubierte für 2 h unter gelegentlichem Mischen bei Raumtemperatur. Der komplette Ansatz (Duolite + Überstand) wurde für die Transformation eingesetzt.

Parallel durchgeführte Versuche, bei denen nicht gebundene Lipolase über Auswaschen aus dem Immobilisat gewonnen wurde, zeigten, dass etwa 80 % der Lipolase trägergebunden vorliegt.

**Durchführung der Versuche:**

10 Ansätze bestehend aus 40 g Sonnenblumenöl und 10 g Ethanol wurden in Bechergläser ausgestattet mit Magnetrührern vorgelegt. Unter Rühren wurden 0,4 g Wasser zugefügt. In die Ansätze 2, 4, 6, 8 und 10 wurden 50 mg festes $Na_2CO_3$ zugegeben. In die Ansätze 1 und 2 wurden 0,2 g Lipolase (Thermomyces lanuginosus Lipase Flüssigpräparation), in die Ansätze 3 und 4 wurden die Dowex Immobilisate, in die Ansätze 5 und 6 wurden die Duolite Immobilisate, in die Ansätze 7 und 8 wurden die MP 1000 Immobilisate und in die Ansätze 9 und 10 wurden die mit $Na_3PO_4$ nachbehandelten MP1000 Immobilisate gegeben. Die Alkoholyse wurde auf einer Multirührplatte bei Raumtemperatur durchgeführt. Die Ansätze 3-10 wurden zweifach durchgeführt. Nach 16 h wurden Proben entnommen und der Gehalt an Glyceriden gaschromatographisch analysiert. Die Auswertung erfolgte über Flächenprozent.

| Ansatz | % Ethylester | Gehalt Monoglycerid | Verhältnis Mono-/Di-/Triglycerid |
|---|---|---|---|
| 1 | 0 | 0% | 0 : 3 : 97 |
| 2 | **56,1** | **28,5 %** | 70 : 30 : 0 |
| 3 (1) | 25,6 | 11,5 % | 16 : 23 : 61 |
| 3 (2) | 26,4 | 10,2 % | 14 : 18 : 68 |
| 4 (1) | 31,6 | 14,1 % | 21 : 36 : 44 |
| 4(2) | 37,9 | 15,7 % | 26 : 30 : 45 |
| 5 (1) | 17,6 | 7,4 % | 9 : 13 : 78 |
| 5 (2) | 22,6 | 9,3 % | 12 : 15 : 73 |
| 6 (1) | 35,5 | 17,1 % | 27 : 34 : 39 |
| 6 (2) | 28,5 | 12,8 % | 18 : 19 : 63 |
| 7(1) | 15,5 | 5,5 % | 7 : 20 : 73 |
| 7 (2) | 24,8 | 8,5 % | 11 : 27 : 61 |
| 8 (1) | 26,1 | 10,5 % | 14 : 37 : 49 |
| 8 (2) | 44,1 | 20,0 % | 36 : 40 : 24 |
| 9 (1) | 24,4 | 9,1 % | 12 : 43 : 45 |
| 9 (2) | 14,2 | 3,5 % | 4 : 13 : 83 |
| 10 (1) | 8,4 | 2,4 % | 3 : 18 : 79 |
| 10 (2) | 15,9 | 4,3% | 5: 14: 81 |

Fazit: Alle Immobilisate mit Lipolase zeigen eine Alkoholyseaktivität. Mit Ausnahme des mit $Na_3PO_4$ vorbehandelten Immobilisats zeigen alle Immobilisate eine zusätzliche Aktivierung durch $Na_2CO_3$. Allerdings ist die Aktivierung der flüssigen Lipolase durch $Na_2CO\mu_3$ wesentlich stärker als die Aktivierung der Immobilisate. Bei gleicher Gesamtenzymeinwaage ist die Alkoholyse mit salzaktivierter Lipolase (Ansatz 2) eindeutig schneller als mit den Immobilisaten.

**Beispiel 4: Umsetzung mit verschiedenen Alkoholen**

Verschiedene Ansätze bestehend aus 40 g Sonnenblumenöl und variablen Mengen verschiedener Alkohole wurden mit Lipolase bei Raumtemperatur einer Alkoholysereaktion unterzogen. Die Ansätze hatten eine Zusammensetzung wie in folgender Tabelle aufgeführt:

| Ansatz | Alkohol | Wasser | Salz | Lipolase |
|---|---|---|---|---|
| 1 | 10 g Ethanol | 0,4 g | 40 mg $Na_3PO_4$ | 0,4 g |
| 2 | 13 g Propanol | 0,4 g | 40 mg $Na_3PO_4$ | 0,4 g |
| 3 | 13 g Isopropanol | 0 g | 40 mg $Na_3PO_4$ | 1,2 g |
| 4 | 16 g Butanol | 0,4 g | 40 mg $Na_3PO_4$ | 0,4 g |
| 5 | 16 g Isobutanol | 0 g | 40 mg $Na_3PO_4$ | 1,2 g |
| 6 | 19 g Isoamylalkohol | 0,4 g | 40 mg $Na_3PO_4$ | 0,8 g |
| 7 | 22 g Hexanol | 0,4 g | 40 mg $Na_3PO_4$ | 0,4 g |

(fortgesetzt)

| Ansatz | Alkohol | Wasser | Salz | Lipolase |
|---|---|---|---|---|
| 8 | 28 g 2-Ethylhexanol | 0,4 g | 40 mg $Na_3PO_4$ | 1,2 g |
| 9 | 7 g Methanol | 0 g | 40 mg $Na_3PO_4$ | 1,2 g |
| 10 | 16 g Butanol | 0 g | 25 mg $Na_2CO_3$ | 1,2 g |
| 11 | 16 g Butanol | 0 g | 50 mg $Na_2CO_3$ | 0,6 g |
| 12 | 16 g Butanol | 0,8g | 50 mg $Na_2CO_3$ | 0,6 g |
| 13 | 23 g Hexanol | 0,8 g | 25 mg $Na_2CO_3$ | 1,2 g |
| 14 | 24 g Hexanol | 2,8 g | 25 mg $Na_2CO_3$ | 1,2 g |
| 15 | 22 g Hexanol | 2,8 g | 50 mg $Na_2CO_3$ | 0,6 g |

Der Gehalt an Glyceriden und Estern wurde gaschromatographisch analysiert. Die Auswertung erfolgte über Flächenprozent, wobei die überschüssigen freien Alkohole nicht mit einberechnet wurden. Proben wurden zu den in der Tabelle angegebenen Zeiten entnommen.

| Ansatz | Dauer [h] | % Alkylester | Gehalt Monoglycerid | Verhältnis Mono-/Di-/Triglycerid |
|---|---|---|---|---|
| 1 | 16 | 59,3 | 26,4 % | 72 : 28 : 0 |
| 2 | 16 | 58,8 | 28,3 % | 74 : 26 : 0 |
| 3 | 16 | 30,6 | 8,7 % | 13 : 55 : 32 |
| 4 | 44 | 42,1 | 17,1 % | 30 : 44 26 |
| 5 | 44 | 41,4 | 17,9 % | 31 : 41 : 28 |
| 6 | 44 | 43,5 | 17,1 % | 31 : 46 : 23 |
| 7 | 44 | 25,1 | 6,9 % | 9 : 36 : 55. |
| 8 | 44 | 27,8 | 14,5 % | 37 : 42 : 20 |
| 9 | 16 | 43,7 | 18,3 % | 34 : 12 : 54 |
| 10 | 40 | 59,7 | 26,3 % | 70 : 30 : 0 |
| 11 | 16 | 57,9 | 26,5 % | 67 : 29 : 4 |
| 12 | 16 | 29,4 | 11,9 % | 17 : 33 : 50 |
| 13 | 40 | 29,3 | 9,2 % | 13 : 43 : 44 |
| 14 | 40 | 69,9 | 19,6 % | 67 : 33 : 0 |
| 15 | 16 | 29,6 | 18,0 % | 26 : 45 : 30 |

Fazit: Eine Alkoholysereaktion wurde mit allen eingesetzten Alkoholen beobachtet. Das Enzym akzeptiert primäre und sekundäre Alkohole sowie geradkettige und verzweigtkettige Alkohole.

Die beste Reaktion wurde mit den Alkoholen Ethanol und Propanol in einem Reaktionsmedium erreicht, dass 2 % Wasser enthielt:

Für die anderen Alkohole müssen die Reaktionsbedingungen zum Teil leicht modifiziert werden um eine optimierte Umsetzung zu erreichen. Detailliertere Untersuchungen mit Butanol (Ansätze 10 - 12) und mit Hexanol (Ansätze 13 - 15) haben gezeigt, dass auch mit diesen Alkoholen die Herstellung von Glyceriden mit einem Monoglyceridgehalt von > 60 % möglich ist. Die Reaktion mit Butanol funktioniert dabei im wasserärmeren Medium besser, wogegen die Reaktion mit Hexanol nur gut in Anwesenheit größerer Wassermengen funktionierte.

Generell kann daraus abgeleitet werden, dass die Konzentration an Wasser erhöht werden muss, wenn der Alkohol hydrophober wird um eine optimale Reaktionsgeschwindigkeit zu erreichen.

**Beispiel 5: Einfluss von Ethanolkonzentration auf Glycerinbildung, Säurebildung und Monoglyceridgehalt**

Verschiedene Ansätze bestehend aus 40 g Sonnenblumenöl und variablen Mengen Ethanol wurden mit jeweils 0,2 g Lipolase bei Raumtemperatur einer Alkoholysereaktion unterzogen. Es wurden jeweils 25 mg $Na_2CO_3$ zugesetzt. Die Ansätze hatten eine Zusammensetzung wie in folgender Tabelle aufgeführt:

| Ansatz | Ethanol | Wasser |
|--------|---------|--------|
| 1 | 15g | 0,2 g |
| 2 | 30 g | 0,2 g |
| 3 | 15g | 0,4 g |
| 4 | 30 g | 0,4 g |
| 5 | 15 g | 0,8 g |
| 6 | 30 g | 0,8 g |

Der Gehalt an Glyceriden wurde gaschromatographisch analysiert. Die Auswertung erfolgte über Flächenprozent. Der Glyceringehalt wurde ebenfalls gaschromatographisch analysiert und ist in nicht kalibrierten Flächenprozent angegeben. Nach Massenbilanz liegen die absoluten Glyceringehalte niedriger, entscheidend hier ist aber der Vergleich der relativen Werte. GC-Proben wurden für die Glycerinbestimmung nach 16 h und für die Glyceridbestimmung nach 40 h Reaktionszeit genommen. Säurezahlen wurden nach 16 h bestimmt.

| Ansatz | Säurezahl | % Glycerin | % Ethylester | % Monoglycerid | Verhältnis Mono-/Di-/Triglycerid |
|--------|-----------|------------|--------------|----------------|----------------------------------|
| 1 | 2 | 1,5 % | 62,2 | 29,2 % | 86 : 14 : 0 |
| 2 | 1 | 0,3 % | 34,5 | 11,4 % | 18 : 35 : 47 |
| 3 | 3 | 2,4 % | 64,3 | 26,2 % | 86 : 14 : 0 |
| 4 | 1 | 0,5 % | 58,9 | 30,6 % | 77 : 23 : 0 |
| 5 | 5 | 2,8 % | 64,7 | 25,8 % | 87 : 13 : 0 |
| 6 | 2 | 1,1 % | 62,4 | 32,2 % | 92 : 8 : 0 |

Da Glycerin bei der verwendeten GC-Methode eine vergleichsweise stärkere Adsorption aufweist als die Ethylester und Glyceride, wurde eine Kalibration direkt in einem Gemisch aus Ethylester, freiem Ethanol und Glyceriden durchgeführt. Die Adsorption über einen Konzentrationsbereich von 0-1,0 Gew.% Glycerin entspricht der Formel:

$$y = 2{,}3x \ (y = \text{Adsorption}, \ x = \text{Einwaage}).$$

Daraus ergibt sich für obige Analytik :

| Ansatz | Glycerin gemessen | Glycerin(Gew. %) nach Kalibration |
|--------|-------------------|-----------------------------------|
| 1 | 1,5 | 0,65 |
| 2 | 0,3 | 0,13 |
| 3 | 2,4 | 1,04 |
| 4 | 0,5 | 0,22 |
| 5 | 2,8 | 1,22 |
| 6 | 1,1 | 0,48 |

Fazit: Je höher die Konzentration an eingesetztem Alkohol ist, desto höhere Monoglyceridgehalte werden erhalten. Bezogen auf die Gesamtglyceride sind Monoglyceridgehalte von über 90 % erreichbar.

Eine Erhöhung des Alkoholgehaltes führte zu einer Reduktion an Nebenproduktbildung wie freie Fettsäure oder Glycerin, das aus der Totalhydrolyse des Öls entsteht.

Die Reaktionsgeschwindigkeit wurde bei einer Erhöhung des Alkoholgehaltes reduziert. Über eine Erhöhung des Wassergehalts konnte die Reaktionsgeschwindigkeit verbessert werden, so dass auch bei einem hohen molaren Überschuss an Ethanol eine gute Monoglyceridbildung erreicht wird (Ansatz 6).

**Beispiel 6: Umsetzung mit verschiedenen Ölen**

In parallelen Ansätzen wird die Hydrolyse mit verschiedenen Ölen untersucht. Jeweils 40 g des Öls werden mit 10 g Ethanol in Bechergläser eingewogen. Unter Rühren wird jeweils 0,4 g Wasser zugegeben und 40 mg festes $Na_3PO_4$ x 12 $H_2O$ wird zugegeben. Die Reaktion wird über Zugabe von 0,4 g Lipolase gestartet. Nach 16 h Reaktionszeit wird eine Probe zur gaschromatographischen Analyse entnommen. Die Auswertung erfolgt über Flächenprozent.

| Ansatz | Öl | % Ethylester | % Monoglycerid | Verhältnis Mono-/Di-/Triglycerid |
|---|---|---|---|---|
| 1 | Sonnenblumenöl | 59,3 | 26,4 % | 72 : 28 : 0 |
| 2 | Rapsöl | 58,7 | 26,4 % | 73 : 27 : 0 |
| 3 | Distelöl | 60,9 | 26,0 % | 76 : 24 : 0 |
| 4 | Sonneblumenöl 2 | 60,0 | 26,7 % | 76 : 24 : 0 |
| 5 | Rizinusöl | 57,5 | 30,0 % | 73 : 27 : 0 |
| 6 | Sojaöl | 60,3 | 26,4 % | 75 : 25 : 0 |
| 7 | Fischöl | 51,0 | 35,0 % | 78 : 22 : 0 |
| 8 | 50 % Rapsöl + 50 % Palmöl | 60,7 | 25,9 % | 75 : 25 : 0 |
| 9 | Speck | 75,4 | 20,7 % | 72 : 28 : 0 |

Fazit: Mit allen eingesetzten Ölen wurde eine gute Alkoholyse beobachtet. Mit allen Ölen wurde ein Monoglyceridanteil von > 70 % bezogen auf die Gesamtglyceride erreicht.

**Beispiel 7: Umsetzung mit verschiedenen alkalischen Salzen**

5 Ansätze mit jeweils 40 g Sonnenblumenöl und 10 g Ethanol wurden eingewogen. Unter Rühren wurde zu allen Ansätzen 0,4 g Wasser gegeben. In Ansatz 1 wurden 40 mg $Na_3PO_4$ x 12 $H_2O$ in Ansatz 2 wurden 11 mg $Na_2CO_3$, in Ansatz 3 wurden 4 mg $Ca(OH)_2$, in Ansatz 4 werden 31 mg Trinatriumcitrat x 2 $H_2O$ gegeben und Ansatz 5 verlief ohne Zusatz eines Salzes. Die Reaktionen wurden über die Zugabe von 0,4 g Lipolase gestartet. Nach 16 h Reaktionszeit wurde eine Probe zur gaschromatographischen Analyse entnommen. Die Auswertung erfolgte über Flächenprozent.

| Ansatz | % Ethylester | Gehalt Monoglycerid | Verhältnis Mono-/Di-/Triglycerid |
|---|---|---|---|
| 1 | 59,3 | 26,4 % | 72 : 28 : 0 |
| 2 | 62,1 | 23,3 % | 74 : 26 : 0 |
| 3 | 50,5 | 28,9 % | 65 : 35 : 0 |
| 4 | 1,0 | 0 % | 0 : 3 : 97 |
| 5 | 0,7 | 0 % | 0 : 2 : 98 |

Fazit: Die Alkoholysereaktion funktionierte gut bei Zugabe von Phosphatsalzen, Carbonatsalzen und Hydroxiden.

**Beispiel 8: Optimierung der Einsatzkonzentration an Salz (für $Na_2CO_3$)**

12 Ansätze mit jeweils 40 g Sonnenblumenöl und 10 g Ethanol wurden eingewogen. Unter Rühren wurde zu den Ansätzen 1 - 6 0,2 g Wasser und zu den Ansätzen 7 -12 0,4 g Wasser gegeben. Unterschiedliche Salzmengen, wie unten in der Tabelle angegeben, wurden hinzugefügt. Die Reaktionen wurden über die Zugabe von 0,2 g Lipolase gestartet. Nach 16 h Reaktionszeit wurde eine Probe zur gaschromatographischen Analyse entnommen. Die Auswertung erfolgte über Flächenprozent.

| Ansatz | $Na_2CO_3$ | % Ethylester | Gehalt Monoglyceride | Verhältnis Mono-/Di-/Triglycerid |
|---|---|---|---|---|
| 1 | 10 mg | 30,0 | 14,7 % | 21 : 32 47 |
| 2 | 25 mg | 53,0 | 29,3 % | 65 : 32 : 3 |
| 3 | 50mg | 54,5 | 30,2 % | 70 : 30 : 0 |
| 4 | 100 mg | 55,9 | 29,1 % | 70 : 30 : 0 |
| 5 | 200 mg | 43,4 | 22,4 % | 41 : 41 : 19 |
| 6 | 500 mg | 4,4 | 0,9 % | 1 : 7 : 92 |
| 7 | 10 mg | 44,2 | 23,5 % | 43 : 38 : 19 |
| 8 | 25 mg | 50,3 | 27,2 % | 56 : 38 : 6 |
| 9 | 50 mg | 55,4 | 30,2 % | 72 : 28 : 0 |
| 10 | 100 mg | 56,9 | 28,5 % | 72: 28 : 0 |
| 11 | 200 mg | 57,2 | 27,5 % | 70 : 30 : 0 |
| 12 | 500 mg | 36,1 | 16,4 % | 26 : 39 : 35 |

Fazit: Eine Erhöhung des Wassergehalts im Ansatz verschiebt das Optimum für die Menge an $Na_2CO_3$ leicht. Bei einer Zugabe von 0,2 g Wasser liegt der Bereich der optimalen Salzmenge zwischen 25 mg und 100 mg, wogegen bei einer Zugabe von 0,4 g Wasser der optimale Bereich zwischen 50 mg und 200 mg liegt.

Zu beachten ist, dass das Optimum an basischem Additiv von der eingesetzten Menge an gepufferter Enzymlösung sowie von der Basenstärke abhängt. Die Versuchsreihe mit $Na_2CO_3$ ist als exemplarisch anzusehen.

**Beispiel 9: Einfluss Temperatur auf die Umesterungsgeschwindigkeit**

6 Ansätze mit jeweils 40 g Sonnenblumenöl und 10 g Ethanol werden eingewogen. Unter Rühren wird zu den Ansätzen 0,4 g Wasser und 50 mg $Na_2CO_3$ zugegeben. Die Reaktionen werden über die Zugabe von 0,2 g Lipolase gestartet. Die Umsetzungen werden bei unterschiedlichen Temperaturen, wie in der Tabelle unten dargestellt, durchgeführt. Nach 24 h Reaktionszeit wird eine Probe zur gaschromatographischen Analyse entnommen. Die Auswertung erfolgt über Flächenprozent.

| Ansatz | Temperatur ˚C | % Ethylester | Gehalt Monoglycerid | Verhältnis Mono-/Di-/Triglycerid |
|---|---|---|---|---|
| 1 | 20 ˚C | 30,0 | 14,7 % | 21 : 32 47 |
| 2 | 25˚C | 53,0 | 29,3 % | 65 : 32 : 3 |
| 3 | 30˚C | 54,5 | 30,2 % | 70 : 30 : 0 |
| 4 | 35C | 55,9 | 29,1 % | 70 : 30 : 0 |
| 5 | 40˚C | 43,4 | 22,4 % | 41 : 41 : 19 |
| 6 | 45˚C | 4,4 | 0,9 % | 1 : 7 : 92 |

Fazit:

Bereits ab Temperaturen oberhalb 30 ˚C wird die Lipase deutlich deaktiviert. Die optimale Reaktionstemperatur liegt im Bereich von 20 - 25 ˚C

**Beispiel 10: Alkoholyse von Sonnenblumenöl und destillative Anreicherung des Monoglycerids**

1,6 kg Sonnenblumenöl und 0,4 kg Ethanol wurden in einen beheizbaren Doppelmantelreaktor eingewogen. Unter Rühren wurden 16 g Wasser und 0,44 g $Na_2CO_3$ zugegeben. Die Reaktion wurde über Zugabe von 8 g Lipolase gestartet und unter Rühren bei Raumtemperatur durchgeführt. Nach 8 h wurden weitere 0,8 kg Ethanol zum Ansatz gegeben. Nach 40 h wurde die Reaktion abgebrochen und eine Probe gaschromatographisch untersucht. Der Reaktionsansatz wird unter Rühren auf 80 ˚C aufgeheizt. Vakuum wird angelegt und das überschüssige Ethanol wurde aus dem Reaktionsansatz verdampft. Danach wurde das Reaktionsgemisch auf Normaldruck entspannt und 16 g Tonsil und 6 g Wasser zugegeben. Die Mischung wurde 30 min bei 80 ˚C gerührt, anschließend eine Stunde unter Vakuum bei 80 ˚C gerührt um Restwasser aus dem Reaktionsansatz zu entfernen. Nach Entspannung des Reaktionsansatzes auf Normaldruck wurde der Ansatz warm filtriert. Eine Probe wurde zur gaschromatographischen Analyse entnommen. Der Ansatz wurde dann über eine Kurzwegdestillation getrennt. Die Reaktionsparameter sind 180 ˚C und 0,5 mbar bei einer Kühlfingertemperatur von 25 ˚C und einer Vorlagetemperatur von 80 ˚C. Eine Massenbilanz der Destillation ergab 29,8 Gew.-% Sumpfprodukt und 70,2 Gew.-% Destillat. Das Monoglycerid enthaltende Sumpfprodukt wurde einer gaschromatographischen Analyse unterzogen.

| Probe | % Ethylester | % Monoglycerid | % Diglycerid | % Triglycerid |
|---|---|---|---|---|
| Nach enz. Reaktion | 61,6 | 30,0 | 4,0 | 0 |
| Nach EtOH Entfernung | 61,4 | 30,5 | 4,0 | 0 |
| Nach Destillation | 3,3 | 81,2 | 14,9 | 0,7 |

| Probe | Verhältnis Ester/Glyceride | Verhältnis Mono-/Di-/Triglycerid |
|---|---|---|
| Nach enz. Reaktion | 64 : 36 | 88 : 12 : 0 |
| Nach EtOH Entfernung | 63 : 37 | 86 : 14 : 0 |
| Nach Destillation | 3 : 97 | 84: 15 : 1 |

**Beispiel 11: Alkoholyse von neuem Sonnenblumenöl und destillative Anreicherung des Monoglycerids**

1,5 kg neues Sonnenblumenöl und 0,75 kg Ethanol wurden in einen beheizbaren Doppelinantelreaktor eingewogen. Unter Rühren wurden 15 g Wasser und 1,5 g $Na_2CO_3$ zugegeben. Die Reaktion wurde über Zugabe von 7,5 g Lipolase gestartet und unter Rühren bei Raumtemperatur durchgeführt. Nach 46 h wurde die Reaktion abgebrochen und eine Probe gaschromatographisch untersucht. Der Reaktionsansätz wurde unter Rühren auf 80 ˚C aufgeheizt. Vakuum wurde angelegt und das überschüssige Ethanol aus dem Reaktionsansatz verdampft. Danach wurde das Reaktionsgemisch auf Normaldruck entspannt und 10 g Tonsil und 6 g Wasser zugegeben. Die Mischung wurde 30 min bei 80 ˚C gerührt, anschließend wurde eine Stunde unter Vakuum bei 80 ˚C gerührt um Restwasser aus dem Reaktionsansatz zu entfernen. Nach Entspannung des Reaktionsansatzes auf Normaldruck wurde der Ansatz warm filtriert. Eine Probe wurde zur gaschromatographischen Analyse entnommen. Der Ansatz wurde dann über eine Kurzwegdestillation getrennt. Die Reaktionsparameter sind 180 ˚C und 0,5 mbar bei einer Kühlfingertemperatur von 25 ˚C und einer Vorlagetemperatur von 80 ˚C. Das Monoglycerid enthaltende Sumpfprodukt wurde einer gaschromatographischen Analyse unterzogen.

| Probe | % Ethylester | % Monoglycerid | % Diglycerid | % Triglycerid |
|---|---|---|---|---|
| Nach enz. Reaktion | 60,5 | 27,6 | 7,1 | 0 |
| Nach EtOH Entfernung | 62,1 | 25,8 | 7,9 | 0 |
| Nach Destillation | 8,0 | 72,0 | 20,0 | 0 |

| Probe | Verhältnis Ester/Glyceride | Verhältnis Mono-/Di-/Triolycerid |
|---|---|---|
| Nach enz. Reaktion | 64 : 36 | 80 : 20 : 0 |

(fortgesetzt)

| Probe | Verhältnis Ester/Glyceride | Verhältnis Mono-/Di-/Triolycerid |
|---|---|---|
| Nach EtOH Entfernung | 65 : 35 | 77 : 23 : 0 |
| Nach Destillation | 8:92 | 78 : 22 : 0 |

**Beispiel 12: Alkoholyse von Distelöl und destillative Anreicherung des Monoglycerids**

1,5 kg Distelöl und 0,75 kg Ethanol wurden in einen beheizbaren Doppelmantelreaktor eingewogen. Unter Rühren wurden 15 g Wasser und 1,5 g $Na_2CO_3$ zugegeben. Die Reaktion wurde über Zugabe von 15g Lipolase gestartet und unter Rühren bei Raumtemperatur durchgeführt. Nach 20 h wurde die Reaktion abgebrochen und eine Probe gaschromatographisch untersucht. Der Reaktionsansatz wurde unter Rühren auf 80 ˚C aufgeheizt. Vakuum wurde angelegt und das überschüssige Ethanol aus dem Reaktionsansatz verdampft. Danach wurde das Reaktionsgemisch auf Normaldruck entspannt und 10 g Tonsil und 6 g Wasser zugegeben. Die Mischung wurde 30 min bei 80 ˚C gerührt, anschließend wurde eine Stunde unter Vakuum bei 80 ˚C gerührt um Restwasser aus dem Reaktionsansatz zu entfernen. Nach Entspannung des Reaktionsansatzes auf Normaldruck wurde der Ansatz warm filtriert. Eine Probe wurde zur gaschromatographischen Analyse entnommen. Der Ansatz wurde dann über eine Kurzwegdestillation getrennt. Die Reaktionsparameter sind 180 ˚C und 0,5 mbar bei einer Kühlfingertemperatur von 25 ˚C und einer Vorlagetemperatur von 80 ˚C. Das Monoglycerid enthaltende Sumpfprodukt wurde einer gaschromatographischen Analyse unterzogen.

| Probe | % Ethylester | % Monoglycerid | % Diglycerid | % Triglycerid |
|---|---|---|---|---|
| Nach enz. Reaktion | 57,0 | 34,2 | 6,7 | 0 |
| Nach EtOH Entfernung | 59,5 | 32,4 | 5,7 | 0 |
| Nach Destillation | 12,4 | 74,0 | 13,6 | 0 |

| Probe | Verhältnis Ester/Glyceride | Verhältnis Mono-/Di-/Triglycerid |
|---|---|---|
| Nach enz. Reaktion | 58 : 42 | 84 : 16 : 0 |
| Nach EtOH Entfernung | 61 : 39 | 85 : 15 : 0 |
| Nach Destillation | 12 : 88 | 84 : 16 : 0 |

**Beispiel 13: Alkoholyse von Rizinusöl**

1,5 kg Rizinusöl und 0,75 kg Ethanol wurden in einen beheizbaren Doppelmantelreaktor eingewogen. Unter Rühren wurden 15 g Wasser und 1,5 g $Na_2CO_3$ zugegeben. Die Reaktion wurde über Zugabe von 15 g Lipolase gestartet und unter Rühren bei Raumtemperatur durchgeführt. Nach 46 h wurde die Reaktion abgebrochen und eine Probe gaschromatographisch untersucht. Der Reaktionsansatz wurde unter Rühren auf 80 ˚C aufgeheizt. Vakuum wurde angelegt und das überschüssige Ethanol aus dem Reaktionsansatz verdampft. Danach wurde das Reaktionsgemisch auf Normaldruck entspannt und 10 g Tonsil und 6 g Wasser zugegeben. Die Mischung wurde 30 min bei 80 ˚C gerührt, anschließend wurde eine Stunde unter Vakuum bei 80 ˚C gerührt um Restwasser aus dem Reaktionsansatz zu entfernen. Nach Entspannung des Reaktionsansatzes auf Normaldruck wurde der Ansatz warm filtriert. Eine Probe wurde zur gaschromatographischen Analyse entnommen.

| Probe | % Ethylester | % Monoglycerid | % Diglycerid | % Triglycerid |
|---|---|---|---|---|
| Nach enz. Reaktion | 59,7 | 33,4 | 5,7 | 0 |
| Nach EtOH Entfernung | 58,8 | 30,0 | 8,9 | 0 |

| Probe | Verhältnis Ester/Glyceride | Verhältnis Mono-/Di-/Triglycerid |
|---|---|---|
| Nach enz. Reaktion | 60 : 40 | 85 : 15 : 0 |

(fortgesetzt)

| Probe | Verhältnis Ester/Glyceride | Verhältnis Mono-/Di-/Triglycerid |
|---|---|---|
| Nach EtOH Entfernung | 60 : 40 | 77 : 23 : 0 |

**Beispiel 14: Untersuchung zur emulgierenden Wirkung der Monoglyceride**

Die emulgierende Eigenschaft der enzymatisch hergestellten und destillierten Monoglyceride wird in einem System aus 80 % Wasser und 20 % Öl untersucht. Als Öle werden Myritol 312 (Medium Chain Triglyceride) und Paraffinöl eingesetzt. Als Vergleichssubstanz dient ein molekulardestilliertes Monoglyceris (Monomuls 90 O) mit einem Monoglyceridgehalt von > 90 %. Die Emulgiereigenschaften im System Myritol 312 / Wasser werden bei Aktivsubstanzkonzentrationen von 1%, 2,5% und 5% bestimmt. Die Emulgiereigenschaften im System Paraffinöl / Wasser werden bei Aktivsubstanzkonzentrationen von 1 %, und 5% bestimmt. Die Art der gebildeten Emulsion wird über Leitfähigkeitsmessung bestimmt. Untersuchungen werden an den Monoglyceridgemischen aus Beispiel 9, 10 und 11 durchgeführt.

Emulsionsbildung im System Myritol 312 / Wasser

| Substanz | Aktivsubstanz | | | Emulsionstyp |
|---|---|---|---|---|
| | 1 % | 2,5 % | 5 % | |
| Monoglycerid (Beispiel 10) | nein | ja | ja | W / O |
| Monoglycerid (Beispiel 11) | nein | nein | ja | W / O |
| Monoglycerid (Beispiel 12) | nein | nein | ja | W / O |
| Monomuls 90 O | nein | ja | ja | W / O |

Emulsionsbildung im System Paraffinöl / Wasser

| Substanz | Aktivsubstanz | | Emulsionstyp |
|---|---|---|---|
| | 1 % | 5 % | |
| Monoglycerid (Beispiel 10) | ja | ja | W / O |
| Monoglycerid (Beispiel 11) | ja | ja | W / O |
| Monoglycerid (Beispiel 12) | ja | ja | W / O |
| Monomuls 90 O | ja | ja | W / O |

Fazit: Alle enzymatisch hergestellten Monoglyceride weisen gute Emulgatoreigenschanen auf. Das Monoglycerid hergestellt aus Sonnenblumenöl hat vergleichbare Emulgatoreigenschaften zum molekulardestillierten Monomuls 90 O. Die Monoglyceride hergestellt aus Neuem Sonnenblumenöl und Distelöl zeigen eine etwas schwächer emulgierende Wirkung, die wahrscheinlich durch die noch erhaltenen höheren Anteile an Ethylester verursacht werden.

**Beispiel 15: Untersuchung der schmierenden Eigenschaften in Dieselkraftstoff**

Die Schmiereigenschaften wurden mit einem HFFR-Test (High Frequency Reciprocating Rig Test) nach CEC Methode F-06-T-94 durchgeführt. Eingesetzt wurden verschiedene Dieselkraftstoffe und Monoglyceridgemische auf Basis Sonnenblumenöl und Rapsöl wie in der unten abgebildeten Tabelle aufgeführt.

| Nummer | Muster | Rohstoff |
|---|---|---|
| Probe 1 | Monoglycerid / Ethylester Gemisch | Sonnenblumenöl |

(fortgesetzt)

| Nummer | Muster | Rohstoff |
|---|---|---|
| Probe 2 | Monoglyceridgemisch destilliert | Rapsöl |
| Probe 3 | Monoglycerid / Butylester Gemisch | Rapsöl |

| | Ester | Monoglycerid | Diglycerid | Triglycerid |
|---|---|---|---|---|
| Probe 1 | 56,0 | 27,8 | 12,8 | < 1 |
| Probe 2 | 3,5 | 61,0 | 32,0 | 2,5 |
| Probe 3 | 66,0 | 21,5 | 9,0 | < 1 |

**Ergebnisse:**

| Nummer | Konzentration in Diesel | HFFR-Wert | Film |
|---|---|---|---|
| Diesel A | Blank | 411 $\mu$m | 19 $\mu$m |
| Probe 1 | 200 ppm | 261 $\mu$m | 67 $\mu$m |
| | | | |
| Diesel B | Blank | 542 $\mu$m | 20 $\mu$m |
| Probe 1 | 100 ppm | 311 $\mu$m | 65 $\mu$m |
| Probe 1 | 150 ppm | 217 $\mu$m | 70 $\mu$m |
| Probe 1 | 200 ppm | 231 $\mu$m | 68 $\mu$m |
| | | | |
| Diesel C | Blank | 615 $\mu$m | |
| Probe 2 | 100 ppm | 183 $\mu$m | |
| Probe 2 | 300 ppm | 170 $\mu$m | |
| Probe 3 | 100 ppm | 279 $\mu$m | |
| Probe 3 | 300 ppm | 195 $\mu$m | |

Fazit: Alle Muster verbessern die Schmiereigenschaften der eingesetzten Dieselkraftstoffe signifikant und senken die HFFR-Werte unter vorgeschriebene Grenzwerte (z.B. aktuell 450 $\mu$m in der Schweiz).

**Beispiel 16: Untersuchung der Fettsäurezusammensetzung des Monoglycerids aus Sonnenblumenöl**

Die Monoglyceridfraktion bzw. die Destillatfraktion aus Beispiel 10 werden nach vollständiger Methylierung mit TMSH in Dichlormethan gaschromatographisch auf ihre Fettsäurezusammensetzung untersucht und gegen das Ausgangsmaterial Sonnenblumenöl verglichen.

Ergebnis:

| | % Sonnenblumenöl | % Monoglycerid | % Destillat |
|---|---|---|---|
| Palmitinsäure | 6,2 | 1,3 | 9,8 |

(fortgesetzt)

|  | % Sonnenblumenöl | % Monoglycerid | % Destillat |
|---|---|---|---|
| Stearinsäure | 3,4 | 1,0 | 3,5 |
| Ölsäure | 25,4 | 23,3 | 25,3 |
| Linolsäure | 65,0 | 74,4 | 61,4 |

Fazit: In der Monoglyceridfraktion sind eine Anreicherung von Linolsäure und eine starke Abreicherung der gesättigten Fettsäuren im Vergleich zum Ausgangsprodukt zu erkennen.

**Patentansprüche**

1. Verfahren zur Herstellung von Monoglyceriden, **dadurch gekennzeichnet, dass** Triglyceride in Anwesenheit von linearen oder verzweigten Alkoholen mit einer Anzahl an Kohlenstoffatomen von 1 - 8 C-Atomen mit einer Lipase aus *Thermomyces lanuginosus* enzymatisch umgesetzt werden, welche durch Zugabe von alkalischen Salzen aktiviert wird.

2. Verfahren nach Anspruch 1, **dadurch gekennzeichnet, dass** in einem weiteren Schritt die Lipase deaktiviert wird.

3. Verfahren nach Anspruch 1 und/oder 2, **dadurch gekennzeichnet, dass** man die Alkoholyse bei Temperaturen von 10˚ bis 40˚ C und einem Wassergehalt von 0,1 - 10 Gew.-% bezogen auf die Menge an Triglycerid durchführt.

4. Verfahren nach einem der Ansprüche 1 bis 3, **dadurch gekennzeichnet, dass** die Lipase in Mengen von 0,05 - 2 % der kommerziell erhältlichen Flüssigpräparation in Bezug auf die Menge an eingesetztem Triglycerid eingesetzt wird.

5. Verfahren nach einem der Ansprüche 1 bis 4, **dadurch gekennzeichnet, dass** es sich bei den alkalischen anorganischen Salzen zur Aktivierung der Lipase um Salze handelt, die ausgewählt sind aus der Gruppe, die gebildet wird von Hydroxiden, Carbonaten und Phosphaten des Natriums, Kaliums, Calciums, Magnesiums und Ammoniums vorgelöst in Wasser.

6. Verfahren nach einem der Ansprüche 1 bis 5, **dadurch gekennzeichnet, dass** die alkalischen anorganischen Salze zur Aktivierung der Lipasen in Mengen zwischen 0,00001 und 1 Gew.-% bezogen auf die Menge an Triglycerid eingesetzt werden.

7. Verfahren nach einem der Ansprüche 1 bis 6, **dadurch gekennzeichnet, dass** Triglyceride aus Fetten und Ölen eingesetzt werden, die einen hohen Anteil an einfach und/oder mehrfach ungesättigter Fettsäuren haben und ausgewählt sind aus der Gruppe, die gebildet wird aus Sonnenblumenöl, Rapsöl, Distelöl, Sojaöl, Leinöl, Erdnussöl, Talge, Olivenöl, Rizinusöl, Palmöl und Altöle wie beispielsweise gebrauchtes Frittierfett.

8. Verfahren nach einem der Ansprüche 1 bis 7, **dadurch gekennzeichnet, dass** als bevorzugte Alkoholkomponente Ethanol oder 1-Propanol eingesetzt werden und der Gehalt an Alkohol 10 bis 75 Gew.-% bezogen auf das Triglycerid beträgt.

9. Verfahren nach einem der Ansprüche 1 bis 8, **dadurch gekennzeichnet, dass** die Alkoholkomponente entfernt wird, bevorzugt über Destillation.

10. Verfahren nach einem der Ansprüche 1 bis 9, **dadurch gekennzeichnet, dass** die Alkylester von den Monoglyceriden getrennt werden, bevorzugt über Destillation.

**Claims**

1. A process for the production of monoglycerides, **characterized in that** triglycerides are enzymatically reacted with a lipase from Thermomyces lanuginosus, which is activated by addition of alkaline salts, in the presence of linear or branched alcohols having a number of carbon atoms of 1 to 8 C atoms.

2. A process as claimed in claim 1, **characterized in that** the lipase is deactivated in a further step.

3. A process as claimed in claim 1 and/or 2, **characterized in that** the alcoholysis is carried out at temperatures of 10˚C to 40˚C and with a water content of 0.1 to 10% by weight, based on the quantity of triglyceride.

4. A process as claimed in any of claims 1 to 3, **characterized in that** the lipase is used in quantities of 0.05 to 2% of the commercially obtainable liquid preparation, based on the quantity of triglyceride used.

5. A process as claimed in any of claims 1 to 4, **characterized in that** the alkaline inorganic salts for activating the lipase are salts selected from the group consisting of hydroxides, carbonates and phosphates of sodium, potassium, calcium, magnesium and ammonium predissolved in water.

6. A process as claimed in any of claims 1 to 5, **characterized in that** the alkaline inorganic salts for activating the lipases are used in quantities of between 0.00001 and 1 % by weight, based on the quantity of triglyceride.

7. A process as claimed in any of claims 1 to 6, **characterized in that** triglycerides from fats and oils which have a high percentage content of mono- and/or polyunsaturated fatty acids and which are selected from the group consisting of sunflower oil, rapeseed oil, thistle oil, soybean oil, linseed oil, peanut oil, tallows, olive oil, castor oil, palm oil and old oils such as, for example, used frying fat are used.

8. A process as claimed in any of claims 1 to 7, **characterized in that** ethanol or 1-propanol is used as the preferred alcohol component and the alcohol content is 10 to 75% by weight, based on the triglyceride.

9. A process as claimed in any of claims 1 to 8, **characterized in that** the alcohol component is removed, preferably by distillation.

10. A process as claimed in any of claims 1 to 9, **characterized in that** the alkyl esters are separated from the monoglycerides, preferably by distillation.

**Revendications**

1. Procédé pour la préparation de monoglycérides, **caractérisé en ce qu'**on transforme enzymatiquement des triglycérides en présence d'alcools linéaires ou ramifiés présentant un nombre d'atomes de 1-8 atomes de C avec une lipase de Thermomyces lanuginosus, qui est activée par l'addition de sels alcalins.

2. Procédé selon la revendication 1, **caractérisé en ce que** la lipase est désactivée dans une autre étape.

3. Procédé selon la revendication 1 et/ou 2, **caractérisé en ce qu'**on réalise l'alcoolyse à des températures de 10˚C à 40˚C et à une teneur en eau de 0,1-10% en poids par rapport à la quantité de triglycéride.

4. Procédé selon l'une quelconque des revendications 1 à 3, **caractérisé en ce que** la lipase est utilisée en des quantités de 0,05-2% de la préparation liquide pouvant être obtenue commercialement par rapport à la quantité de triglycéride utilisé.

5. Procédé selon l'une quelconque des revendications 1 à 4, **caractérisé en ce qu'**il s'agit, pour les sels inorganiques alcalins destinés à l'activation de la lipase de sels qui sont choisis dans le groupe formé par les hydroxydes, les carbonates et les phosphates du sodium, du potassium, du calcium, du magnésium et de l'ammonium, dissous au préalable dans l'eau.

6. Procédé selon l'une quelconque des revendications 1 à 5, **caractérisé en ce que** les sels inorganiques alcalins destinés à l'activation des lipases sont utilisés en des quantités entre 0,00001 et 1% en poids par rapport à la quantité de triglycéride.

7. Procédé selon l'une quelconque des revendications 1 à 6, **caractérisé en ce qu'**on utilise des triglycérides provenant de graisses et d'huiles qui présentent une proportion élevée d'acides gras monoinsaturés et/ou polyinsaturés et sont choisis dans le groupe formé par l'huile de tournesol, l'huile de colza, l'huile de chardon, l'huile de soja, l'huile de lin, l'huile d'arachide, les suifs, l'huile d'olive, l'huile de ricin, l'huile de palme et les huiles usagées, telles que par exemple les graisses de friture usagées.

8. Procédé selon l'une quelconque des revendications 1 à 7, **caractérisé en ce qu'**on utilise comme composant alcoolique préféré l'éthanol ou le 1-propanol et la teneur en alcool est de 10 à 75% en poids par rapport au triglycéride.

9. Procédé selon l'une quelconque des revendications 1 à 8, **caractérisé en ce que** le composant alcoolique est éliminé, de préférence par distillation.

10. Procédé selon l'une quelconque des revendications 1 à 9, **caractérisé en ce que** les esters d'alkyle des monoglycérides sont séparés, de préférence par distillation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 9013656 A
- WO 9004033 A
- US 5935828 A
- US 5316927 A
- WO 9116441 A
- WO 9116442 A
- US 5116745 A
- EP 407959 A

- WO 0206505 A
- JP 60102192 B
- JP 03187385 B
- JP 03103499 B
- EP 0721492 A
- WO 0119941 A
- WO 0063322 A

### In der Beschreibung aufgeführte Nicht-Patentliteratur

- Recent Res. Devel. Oil Chem. 1999, vol. 3, 93-106

- Hydrolases in Organic Synthesis. Wiley-VCH, 1999
- *Recent Res. Devel. Oil Chem.,* 1999, vol. 3